# EUROPÄISCHE PATENTSCHRIFT

(11) **EP 3 018 195 B1**
(45) Veröffentlichungstag und Bekanntmachung des Hinweises auf die Patenterteilung: **31.05.2017**
(21) Anmeldenummer: 15192541.9
(22) Anmeldetag: 02.11.2015
(51) Int. Cl.: C12M 3/00, C12M 1/00, C12M 1/42, C12M 1/34, A01N 1/02

(54) **BIOREAKTORSYSTEM UND VERFAHREN ZUR LANGZEITSTABILISIERUNG VON KORNEAGEWEBE**
BIOREACTOR SYSTEM AND METHOD FOR LONG-TERM STABILITY OF CORNEA TISSUE
SYSTÈME DE BIORÉACTEUR ET PROCÉDÉ DE STABILISATION À LONG TERME DE TISSU CORNÉEN

(30) Priorität: 05.11.2014 DE 102014222547
(43) Veröffentlichungstag der Anmeldung: 11.05.2016
(73) Patentinhaber: Fraunhofer-Gesellschaft zur Förderung der angewandten Forschung e.V., 80686 München (DE)
(72) Erfinder: Groeber, Florian, 97072 Würzburg (DE); Hansmann, Jan, 97246 Eibelstadt (DE); Walles, Heike, 97082 Würzburg (DE); Rossi, Angela, 97072 Würzburg (DE); Schwarz, Thomas, 97286 Sommerhausen (DE)
(74) Vertreter: Schrell, Andreas

(56) Entgegenhaltungen:
- WO-A1-2006/052478
- WO-A1-2014/140434
- US-A- 4 844 242
- US-A- 5 789 240
- US-A1- 2011 014 690
- US-A1- 2014 017 770

## Beschreibung

Die vorliegende Erfindung betrifft ein Bioreaktorsystem zur Kultivierung, insbesondere Lagerung, und Herstellung von Hornhautimplantaten sowie Verfahren zur Kultivierung, insbesondere Lagerung, und Herstellung von Hornhautimplantaten sowie Verfahren zur Testung von Agenzien an Hornhäuten.

Die Hornhautimplantation ist die am häufigsten durchgeführte Form der Organ- bzw. Gewebeverpflanzung. Der Bedarf an Hornhautimplantaten in Deutschland liegt bei etwa 7000 Hornhäuten im Jahr, tatsächlich stehen aber nur etwa 4000 Hornhautimplantate zur Verfügung. Im Gegensatz zu anderen Organimplantaten können Hornhautimplantate bis zu 4 Wochen gelagert werden. Um die Funktion der Hornhaut während dieses Zeitraums zu erhalten und zu garantieren, dass dabei kein Schaden am Gewebe entsteht, ist eine Lagerung nahe den physiologischen Bedingungen sehr wichtig. Durch das Fehlen adäquater technischer Systeme zur Lagerung ist der Lagerungszeitraum bisher auf 4 Wochen beschränkt. Ältere Hornhäute verlieren ihre Funktionalität und stehen nicht mehr als Implantat zur Verfügung. Dies ist einer der Gründe für den oben beschrieben Mangel an Spenderhornhäuten.

Gegenwärtig sind verschiedene Stabilisierungsverfahren bekannt. So war es bis Mitte der 1980 Jahre üblich, Spendern von Hornhäuten den kompletten Augapfel zu entnehmen und für maximal 4 Tage in einer feuchten Kammer aufzubewahren. Diese Methodik wurde durch die Organkultur der Spenderhornhäute abgelöst. Dabei wird die korneosklerale Scheibe aus dem Auge der Leiche präpariert, im Kulturmedium zur Hornhautbank transportiert und dort in einer Zellkulturflasche bis zu vier Wochen vital gehalten.

Aus der WO 20141/40434 ist eine medizinische Vorrichtung zur Langzeitlagerung eines Cornea-Implantats bekannt. Die Vorrichtung weist ein Bodenteil und ein Deckelelement für die Augapfel-abgewandte Außenseite der Hornhaut (endothel) für die Innenseite der Hornhaut) sowie eine umlaufende Seitenwand auf. Aus der US 4,844,242 sind Aufbewahrungsbehälter für Hornhäute bekannt, in denen Hornhäute an der Unterseite der Behälterdeckel fixiert und so in die Aufbewahrungsbehälter eingesteckt werden.

Aus der US 2011/0014690 A1 sind Aufbewahrungsbehälter für Hornhäute bekannt, die Medienaustausche zwischen der Ober- und Unterseite der Hornhaut erlauben.

Aus der WO 2006/052478 A1 sind Verfahren bekannt, in denen Hornhäute auf eine Plattform gelegt und mit einer Klemme fixiert werden. Die so fixierten Hornhäute werden in einem Aufbewahrungsbehälter, der mit Medium gefüllt ist, aufbewahrt.

Alle diese Verfahren und Aufbewahrungsbehälter sind jedoch vergleichsweise einfacher Struktur und erlauben bestenfalls eine kurzzeitige Kultivierung unter statischen Bedingungen, wobei die Funktionalität der Hornhäute nicht oder lediglich über einen kurzen Zeitraum gewährleistet wird. Nach wie vor besteht daher der Bedarf an Vorrichtungen und Kultivierverfahren, die es ermöglichen, Hornhäute möglichst lange aufzubewahren und gleichzeitig eine hohe Funktionalität auch nach längerer Lagerung zu gewährleisten.

Das der vorliegenden Erfindung zu Grunde liegende technische Problem liegt also insbesondere darin, Vorrichtungen und Verfahren bereitzustellen, die die vorgenannten Nachteile überwinden, insbesondere eine möglichst über einen langen Zeitraum andauernde Aufbewahrung von Hornhautimplantaten bei gleichzeitig geringem oder gar nicht vorkommenden Funktionsverlust zu gewährleisten.

Das der vorliegenden Erfindung zu Grunde liegende technische Problem wird durch die Lehre der unabhängigen Ansprüche gelöst.

Die vorliegende Erfindung löst das ihr zu Grunde liegende technische Problem insbesondere durch ein Bioreaktorsystem zur Kultivierung von Hornhautimplantaten, umfassend
a) eine Kulturkammer mit einem Deckelelement, mindestens einer Seitenwand, mindestens einem an der Seitenwand oder dem Deckelelement angeordneten Tropf- oder Zerstäuberapplikator, mindestens einer Auslassöffnung und einem Bodenteil, welches Bodenteil eine zum Deckelelement hin zumindest bereichsweise gewölbte Oberfläche und mindestens eine erste und eine zweite Durchlassöffnungen aufweist,
b) mindestens eine Flüssigkeitspumpe,
c) ein erstes und zweites, jeweils mit der mindestens einen Flüssigkeitspumpe in Verbindung stehendes Flüssigkeitskreislaufsystem, wobei das erste Flüssigkeitskreislaufsystem den mindestens einen Tropf- oder Zerstäuberapplikator mit der mindestens einen Auslassöffnung und das zweite Flüssigkeitskreislaufsystem die erste mit der zweiten Durchlassöffnung verbindet und
d) mindestens eine in der Kulturkammer relativ zur Seitenwand beweglich angeordnete Fixiervorrichtung zur vollumfänglichen Fixierung des Peripheriebereiches eines parallel zum Bodenteil angeordneten Hornhautimplantats an der mindestens einen Seitenwand oder einem Randbereich des Bodenteils, wobei durch die Fixierung des Peripheriebereichs des Hornhautimplantats eine fluiddichte Abtrennung des ersten Flüssigkeitskreislaufsystems vom zweiten Flüssigkeitskreislaufsystem erreicht wird.

Die vorliegende Erfindung stellt daher ein Bioreaktorsystem zur Kultivierung von Hornhautimplantaten bereit, welcher eine Kulturkammer umfasst. Diese Kulturkammer ist aus mindestens einer Seitenwand, einem Bodenteil sowie einem der Kulturkammer zugeordneten, diese nach oben reversibel abschließenden Deckelelement aufgebaut.

Sofern die Kulturkammer in Aufsicht gesehen eine kreisrunde Grundform hat, weist diese eine den gesamten Umfang der Kulturkammer begrenzende Seitenwand auf. Sofern die Kulturkammer in Aufsicht gesehen eine eckige Grundform hat, weist sie eine der Anzahl der Ecken entsprechende Zahl von den gesamten Umfang der Kulturkammer begrenzenden Seitenwänden auf.

Der Bodenteil grenzt die Kulturkammer nach unten hin ab und weist um seinen gesamten Umfang gesehen einen Randbereich auf, der unmittelbar an die mindestens eine Seitenwand angrenzt und einen Zentralbereich des Bodenteils allseits umschließt. Im Bereich des Randbereiches sind keine Durchlassöffnungen angeordnet. Die Durchlassöffnungen befinden sich im Zentralbereich des Bodenteils. Im Zusammenhang mit der vorliegenden Erfindung wird unter dem Randbereich des Bodenteils der unmittelbar an die Seitenwand angrenzende periphere, das heißt randwärts gelegene, Bereich des Bodenteils verstanden, in dem sich keine Durchlassöffnungen befinden.

Im Zusammenhang mit der vorliegenden Erfindung wird unter dem Zentralbereich des Bodenteils der von dem Randbereich des Bodenteils umschlossene, zentral gelegene Bereich des Bodenteils verstanden, in welchem die mindestens zwei Durchlassöffnungen angeordnet sind und der auf seiner nach oben, das heißt zum Deckelelement hin, gewandten Seite vorzugsweise vollständig oder teilweise gewölbt ist. Der Zentralbereich des Bodenteils der Kulturkammer ist also zumindest bereichsweise, vorzugsweise in seinem Zentrum, hier auch als Kernbereich bezeichnet, gewölbt, weist also eine bereichsweise gewölbte Oberfläche und mindestens zwei Durchlassöffnungen auf, wobei diese sich im gewölbten oder nicht gewölbten Bereich des Zentralbereichs befinden können. Die Unterseite des Bodenteils ist vorzugsweise eben.

Das Deckelelement verschließt die Kulturkammer nach oben, vorzugsweise in reversibler Art. Das Deckelelement kann beispielsweise auflegbar, steckbar oder schraubbar mit der Kulturkammer reversibel zu verbinden sein oder als Klappdeckel ausgeführt sein. Die die Kulturkammer bildende mindestens eine Seitenwand und das Bodenteil sind in bevorzugter Ausführungsform einstückig ausgeführt.

In der Kulturkammer befindet sich eine relativ zur Seitenwand beweglich angeordnete Fixiervorrichtung, die dazu dient, ein Hornhautimplantat in der Kulturkammer in paralleler Ausrichtung zum Bodenteil zu fixieren. Die Fixierung wird erfindungsgemäß dadurch bereitgestellt, dass die Fixiervorrichtung so ausgebildet ist, dass sie die peripheren Randbereiche des Hornhautimplantats, bevorzugt den Sklera-Rand, über den gesamten Umfang des Hornhautimplantats erfasst und in der Kulturkammer fixieren kann. Die Fixiervorrichtung ist insbesondere so ausgebildet und angeordnet, dass sie eine zu der Oberfläche des Bodenteils im Wesentlichen parallele, insbesondere Ebenen-parallele, Positionierung des Hornhautimplantats auf oder oberhalb der Oberfläche des Bodenteils erlaubt. Die, vorzugsweise als Hohlzylinder, insbesondere Ring, ausgebildete Fixiervorrichtung kann insbesondere so ausgebildet sein, dass sie das Hornhautimplantat um dessen Gesamtumfang an der mindestens einen Seitenwand über den gesamten Umfang des Kulturkammerinnenraums oder auf dem Randbereich des Bodenteils um dessen gesamten Umfang fluiddicht fixiert, zum Beispiel festklemmt.

In besonders bevorzugter Ausführungsform kann vorgesehen sein, dass die Fixiervorrichtung ein Hohlzylinder mit entlang seiner Längsachse gesehen U-förmigem Profil ist, also ein Hohlzylinder mit Seitenwand und Bodenteil, wobei der Zentralbereich des Bodenteils eine Öffnung aufweist, insbesondere eine Öffnung, die in Form, Größe und Position der Form, Größe und Position der gewölbten Oberfläche des Bodenteils entspricht.

Sofern in dem erfindungsgemäßen Bioreaktorsystem in betriebsbereitem Zustand ein Hornhautimplantat eingebracht und fluiddicht fixiert wurde, unterteilt dieses Hornhautimplantat die Kulturkammer in einen oberhalb des Hornhautimplantats und einen unterhalb des Hornhautimplantats ausgebildeten oberen und unteren Kulturkammerinnenraum. Das Hornhautimplantat bildet, sofern in das erfindungsgemäße Bioreaktorsystem eingebracht und fixiert, eine fluiddichte Abtrennung zwischen oberem und unterem Kulturkammerinnenraum aus.

Das erfindungsgemäße Bioreaktorsystem weist zudem mindestens eine Flüssigkeitspumpe auf, die mit einem ersten und zweiten Flüssigkeitskreislauf in Fluidverbindung steht.

Der erste Flüssigkeitskreislauf umfasst mindestens eine Fluidverbindung, insbesondere Leitung oder Kanal, von der Flüssigkeitspumpe zu dem mindestens einen im Deckelelement oder einer Seitenwand angeordneten Tropf- oder Zerstäuberapplikator, einen oberen Kulturkammerinnenraum, der sich oberhalb eines in der Kulturkammer in betriebsbereitem Zustand fixierten Hornhautimplantats befindet und mindestens eine Fluidverbindung, insbesondere Leitung oder Kanal, von der mindestens einen Auslassöffnung des oberen Kulturkammerinnenraums zu der Flüssigkeitspumpe.

Die Auslassöffnung der Kulturkammer kann derart im Randbereich des Bodenteils oder vorzugsweise im unteren Bereich der Seitenwand der Kulturkammer, angeordnet sein, dass sie in Fluidverbindung mit dem oberen Kulturkammerinnenraum steht und sich demgemäß, sofern sich im betriebsbereiten Zustand ein fluiddicht fixiertes Hornhautimplantat in der Kulturkammer befindet, keine Fluidverbindung zwischen dem ersten und zweiten Flüssigkeitskreislauf, zwischen dem oberen und unteren Kulturkammerinnenraum und zwischen den Durchlassöffnungen im Bodenteil und der Auslassöffnung im oberen Kulturkammerinnenraum ergibt.

In einer bevorzugten Ausführungsform der vorliegenden Erfindung kann vorgesehen sein, dass die in der Kulturkammer vorgesehene Auslassöffnung mit einer in der Seitenwand oder in dem vorzugsweise vorhandenen Bodenteil der Fixiervorrichtung vorhandenen Öffnung in Fluidverbindung steht, sodass durch die in der Fixiervorrichtung entweder in dessen Seitenwand oder in dessen Bodenteil vorhandene Öffnung Kulturmedium aus dem oberen Kulturkammerinnenraum in die Leitung des ersten Flüssigkeitskreislaufes fließen kann, ohne dass eine Vermischung mit dem zweiten Flüssigkeitskreislauf auftritt.

Der zweite, mit der mindestens einen Flüssigkeitspumpe verbundene Flüssigkeitskreislauf umfasst mindestens eine Fluidverbindung, insbesondere Leitung oder Kanal, von der Flüssigkeitspumpe zu einer ersten Durchlassöffnung im Bodenteil, die als Einlassöffnung fungiert, einen unteren Kulturkammerinnenraum, der sich unterhalb eines in der Kulturkammer in betriebsbereitem Zustand fixierten Hornhautimplantats befindet und mindestens eine Fluidverbindung, insbesondere Leitung oder Kanal, von der zweiten Durchlassöffnung im Bodenteil, die als Auslassöffnung fungiert, zu der Flüssigkeitspumpe zurück.

Auf diese Art und Weise wird erfindungsgemäß erreicht, dass, sobald in betriebsbereitem Zustand ein Hornhautimplantat in dem erfindungsgemäßen Bioreaktorsystem eingebracht und fixiert ist, zwei voneinander getrennte Flüssigkeitskreisläufe vorliegen. Die Trennung der beiden Flüssigkeitskreisläufe wird dadurch realisiert, dass die Fixiervorrichtung das naturgemäß fluiddichte Hornhautimplantat so an dem Bodenteil oder der mindestens einen Seitenwand fluiddicht fixiert, dass ein Flüssigkeitsaustausch zwischen dem ersten und zweiten Kulturkammerinnenraum und damit zwischen dem ersten und zweiten Flüssigkeitskreislaufsystem nicht möglich ist. Die fluiddichte Fixierung kann in bevorzugter Ausführung dadurch realisiert werden, dass die Fixiervorrichtung den Peripheriebereich des Hornhautimplantats vollumfänglich auf den entsprechenden Randbereich der Oberfläche des Bodenteils fluiddicht drückt, sodass ein die beiden Durchlassöffnungen im Zentralbereich des Bodenteils durchfließender Mediumstrom lediglich oberhalb des Bodenteils im unteren Kulturkammerinnenraum und damit unterhalb des Hornhautimplantates hindurch fließen kann, jedoch nicht in den Bereich oberhalb des Hornhautimplantats, das heißt den oberen Kulturkammerinnenraum, gelangt, welcher dem ersten Flüssigkeitskreislauf zugeordnet ist. Das in betriebsbereitem Zustand in das erfindungsgemäßeBioreaktorsystem eingebrachte und dort fixierte Hornhautimplantat stellt daher in betriebsbereitem Zustand die fluiddichte Abtrennung zwischen erstem und zweitem Flüssigkeitskreislauf dar.

Die erfindungsgemäß vorgesehene Eignung einer Fixiervorrichtung zur vollumfänglichen Fixierung des Peripheriebereiches eines parallel zum Bodenteil angeordneten Hornhautimplantates an der mindestens einen Seitenwand oder einem Randbereich des Bodenteils, wobei durch die Fixierung des Peripheriebereichs des Hornhautimplantats eine fluiddichte Abtrennung des ersten von einem zweiten Flüssigkeitskreislaufsystem erreicht wird, bedeutet erfindungsgemäß nicht, dass ein Bioreaktorsystem der erfindungsgemäßen Art ein Hornhautimplantat enthalten muss. Die erfindungsgemäß eingesetzte Fixiervorrichtung definiert sich durch Angabe der Eignung und Funktion zur Fixierung eines Hornhautimplantates in ihrer konstruktiven Ausgestaltung und Anordnung, nicht aber zwangsläufig dadurch, dass in der erfindungsgemäßen Vorrichtung ein Hornhautimplantat als konstitutiver Bestandteil vorhanden sein muss.

Die vorliegende Erfindung betrifft daher in besonders bevorzugter Ausführungsform ein Bioreaktorsystem der beschriebenen Form ohne dass ein Hornhautimplantat in diesen eingebracht ist. In einer weiteren Ausführungsform betriff die vorliegende Erfindung auch ein Bioreaktorsystem der vorliegenden Erfindung, der ein Hornhautimplantat enthält.

Das erfindungsgemäße Bioreaktorsystem erlaubt in vorteilhafter und überraschender Weise eine Langzeit-Lagerung von implantierbaren und/oder transplantierbaren Hornhäuten über das erfindungsgemäße fluidische System, welches eine in-vivo-Situation von Hornhäuten simuliert. Insbesondere erlaubt es das erfindungsgemäße Bioreaktorsystem, die Qualität und Lebensdauer von Hornhautimplantaten signifikant zu erhöhen. Das erfindungsgemäße Bioreaktorsystem erlaubt es, explantierte Hornhäute in die Kulturkammer einzubringen und dort mittels einer Fixiervorrichtung, insbesondere Hohlzylinder, zum Beispiel mittels eines Ringes, zu fixieren. Der erfindungsgemäße Bioreaktor kann in steriler Weise betrieben werden und garantiert daher sterile Bedingungen für die Kultivierung. Das erfindungsgemäße Bioreaktorsystem kann in Standard-Zellkulturinkubatoren betrieben werden. In dem erfindungsgemäßen Bioreaktorsystem generiert mindestens eine Flüssigkeitspumpe in betriebsbereitem Zustand zwei getrennte Flüssigkeitskreisläufe für die Oberseite und Unterseite des Hornhautimplantats. Erfindungsgemäß ist es dadurch ermöglicht, jeweils den beiden physiologisch verschiedenen Hornhautseiten passende Bedingungen getrennt in zwei Kompartimenten der Kulturkammer einzustellen.

So ist es in einer besonders bevorzugten Ausgestaltung der vorliegenden Erfindung möglich, mit dem zweiten Flüssigkeitskreislaufsystem die Hornhaut-Unterseite im unteren Kulturkammerinnenraum kontinuierlich mit einem speziellen laminar fließenden Kulturmedium, insbesondere Nährmedium, zu umspülen und dabei in bevorzugter Ausführungsform einen zusätzlichen hydrostatischen Druck aufzubauen, beispielsweise über eine Steuerung der Pumpleistung oder einen Strömungswiderstand. Eine derartig bewirkte mechanische Belastung stellt einen wichtigen Stimulus für das Zellverhalten von Hornhäuten dar. So haben experimentelle Studien ermittelt, dass Fibroblasten der Hornhaut sehr schnell auf eine erhöhte und auch verringerte mechanisch Belastung der extrazellulären Matrix reagieren, um das Spannungsgleichgewicht zu erhalten und sich entlang der mechanischen Belastung auszurichten. Das erfindungsgemäße Bioreaktorsystem kann so reguliert werden, dass durch den zweiten Flüssigkeitskreislauf ein Druck von beispielsweise 5 bis 40 mm Hg, vorzugsweise 7 bis 35 mm Hg, insbesondere 10 bis 20 mm Hg, an der Hornhaut-Unterseite eingestellt wird, was dem physiologischen Augeninnendruck entspricht. Außerdem wird so der Innendruck der Hornhaut während der Kultivierung in natürlicher Form belassen.

Darüber hinaus ist es erfindungsgemäß möglich, mit dem ersten Flüssigkeitskreislaufsystem die Hornhaut-Oberseite zyklisch mit einem Kulturmedium, insbesondere flüssigem Kulturmedium, insbesondere Nährmedium, das in bevorzugter Ausführungsform der Tränenflüssigkeit entspricht oder Tränenflüssigkeit ist, zu umspülen. Unter in-vivo-Bedingung ist die Hornhaut-Oberseite nämlich regelmäßig Luft ausgesetzt. Bei Kultivierungsbedingungen gemäß dem Stand der Technik sind Epithelzellen jedoch zu jedem Zeitpunkt mit Medium bedeckt, was nicht den physiologischen Bedingungen entspricht. Das erfindungsgemäße Bioreaktorsystem dagegen ermöglicht eine regelmäßige Benetzung der Hornhaut durch die in dem oberen Kulturkammerinnenraum angeordneten Applikator, was dem natürlichen Benetzen durch den Wimpernschlag entspricht. Der erste Flüssigkeitskreislauf führt Nährmedium in den Applikator und lässt ihn entweder periodisch in geringer Menge auf die Hornhaut-Oberseite tropfen oder vernebelt periodisch oder konstant die Mediumsflüssigkeit. Die erfindungsgemäß bevorzugte periodische Applikation eines Nährmediums im ersten Flüssigkeitskreislauf kann entweder über einen Strömungswiderstand oder über die Steuerung der Pumpleistung erreicht werden.

Die insbesondere in betriebsbereitem Zustand durch das Hornhautimplantat in zwei Innenräume, nämlich einen oberen und einen unteren Kulturkammerinnenraum, getrennte Kulturkammer kann erfindungsgemäß aktiv perfundiert werden. Unter einer aktiven Perfusion wird im Zusammenhang mit der vorliegenden Erfindung insbesondere verstanden, dass durch die mindestens eine Flüssigkeitspumpe wenigstens eine Strömung, insbesondere eine erste Strömung in einem ersten Flüssigkeitskreislauf und eine zweite Strömung in einem zweiten Flüssigkeitskreislauf oder lediglich eine Strömung, zum Beispiel in dem zweiten Flüssigkeitskreislauf, aufgebaut wird, die vorzugsweise parallel zu dem Bodenteil und insbesondere bevorzugt parallel zu der Unterseite eines in der Kulturkammer angeordneten Hornhautimplantats verläuft. Durch einen Strömungsverlauf dieser Art, insbesondere parallel zu dem Hornhautimplantat, ist es erfindungsgemäß möglich, auf gezielte Art und Weise das Hornhautimplantat Scherkräften in Form eines "shear flow" oder "shear stress" auszusetzen. Insbesondere ist es in bevorzugter Ausführungsform möglich, in dem ersten oder zweiten, insbesondere dem zweiten, Flüssigkeitskreislauf eine Modulation der Förderrate und Druckkurve zu erreichen, um so die physiologischen Verhältnisse der in-vivo-Situation möglichst genau im vorliegenden Bioreaktorsystem wiederzugeben.

Erfindungsgemäß wird es durch die Bereitstellung von zwei Flüssigkeitskreisläufen erreicht, dass in jedem Innenraum der Kulturkammer jeweils spezifische Kulturbedingungen, insbesondere Druck- und Flussbedingungen und Scherkräfte des eingesetzten Kulturmediums, und gegebenenfalls darin eingesetzter unterschiedlicher oder gleicher chemischer oder biologischer Agenzien eingestellt werden können, die individuell in dem oberen und unteren Kulturkammerinnenraum ein gewünschtes Kulturprofil einstellbar machen. Unter einem Kulturprofil wird das Einstellen einer bestimmten Fließgeschwindigkeit und eines bestimmten Pumpvolumen des Kulturmediums verstanden, um dadurch konkret und genau definierte Scherkräfte auf das Hornhautimplantat zu bewirken. Insbesondere ist es erfindungsgemäß also möglich, in den Kulturkammerinnenräumen unabhängig voneinander physiologische Bedingungen, zum Beispiel in Form von unterschiedlichen Flüssigkeitszusammensetzungen Temperaturen oder Scherkräften, herzustellen.

Im Zusammenhang mit der vorliegenden Erfindung wird unter der Hornhaut, auch als Kornea bezeichnet, der glasklare, im in-vivo-Zustand von Tränenflüssigkeit benetzte, gewölbte vordere Teil der äußeren Augenhaut verstanden. In besonders bevorzugter Ausführungsform erfasst der Begriff "Hornhaut" im Zusammenhang mit der vorliegenden Erfindung auch den die Hornhaut zirkulär umschließenden Limbus, das heißt die Übergangszone zwischen Hornhaut im engeren Sinn und Lederhaut, auch als Sklera bezeichnet, des Augapfels.

In einer weiteren bevorzugten Ausführungsform wird im Zusammenhang mit der vorliegenden Erfindung mit dem Begriff "Hornhaut" auch eine Hornhaut erfasst, die die Hornhaut, den diese Hornhaut zirkulär umfassenden Limbus und darüber hinaus einen Lederhaut-Rand, das heißt Sklera-Rand, umfasst, welcher beispielsweise von 1 bis 8 mm, vorzugsweise 1 bis 7 mm, bevorzugt 1 bis 6 mm, bevorzugt 1 bis 5 mm, bevorzugt 2 bis 5 mm, bevorzugt 1 bis 4 mm, bevorzugt 3 bis 4 mm, bevorzugt 1 bis 3 mm, bevorzugt 1 bis 2 mm breit ist. Sofern eine erfindungsgemäß eingesetzte Hornhaut einen Sklera-Rand aufweist, liegt auch ein Limbus vor.

Im Zusammenhang mit der vorliegenden Erfindung ist eine Hornhaut vorzugsweise eine menschliche Hornhaut, kann jedoch auch eine tierische Hornhaut sein. Im Zusammenhang mit der vorliegenden Erfindung ist eine Hornhaut in Aufsicht gesehen elliptisch oder kreisrund. Im Zusammenhang mit der vorliegenden Erfindung weist eine Hornhaut, insbesondere eine menschliche Hornhaut, einen Durchmesser von 8 bis 14 mm, vorzugsweise 10 bis 12 mm, vorzugsweise 7 bis 13 mm, vorzugsweise 9 bis 11 mm, auf.

Im Zusammenhang mit der vorliegenden Erfindung wird unter einem "Hornhautimplantat" eine künstlich hergestellte oder natürliche, insbesondere eine einem humanen oder tierischen Spender entnommene, Hornhaut verstanden. In bevorzugter Ausführung der Erfindung kann diese einem Empfänger, insbesondere operativ, zugeführt oder zu Testzwecken verwendet werden. Im Zusammenhang mit der vorliegenden Erfindung stammt eine für die vorliegende Erfindung einsetzbare, das heißt zu kultivierende, natürliche Hornhaut aus einem lebenden oder vorzugsweise toten Spender, insbesondere ist die Hornhaut eine innerhalb von 72 Stunden nach dem Ableben des Spenders entnommene Hornhaut.

In besonders bevorzugter Ausführungsform ist ein erfindungsgemäß einsetzbares Hornhautimplantat eine einen vorzugsweise 1 bis 8 mm, vorzugsweise 1 bis 6 mm, vorzugsweise 2 bis 5 mm oder 3 bis 4 mm breiten Sklera-Rand aufweisende Hornhaut.

Im Zusammenhang mit der vorliegenden Erfindung wird unter einem Peripheriebereich eines Hornhautimplantates der um den gesamten Umfang des Hornhautimplantates vorhandene Hornhautrand verstanden, der von der erfindungsgemäß vorgesehenen Fixiervorrichtung erfasst und an der Seitenwand oder dem Bodenteil der erfindungsgemäß bereitgestellten Kulturkammer fixiert wird.

Vorzugsweise ist der Peripheriebereich des Hornhautimplantats ein Sklera-Rand eines Hornhautimplantats.

Im Zusammenhang mit der vorliegenden Erfindung wird ein synthetisches Hornhautimplantat auch als Hornhautäquivalent bezeichnet.

Im Zusammenhang mit der vorliegenden Erfindung wird unter einem Bioreaktorsystem insbesondere ein Gefäß, vorzugsweise ein Kultivierungsgefäß mit für den Betrieb als Kultivierungssystem erforderlichen weiteren Elementen wie Flüssigkeitskreisläufen und mindestens einer Pumpe, verstanden, das dazu bestimmt und geeignet ist, eine Hornhaut oder ein Hornhautgrundgerüst zu kultivieren, insbesondere zu lagern.

Im Zusammenhang mit der vorliegenden Erfindung wird unter dem Begriff "Kultivierung" die Schaffung und insbesondere Aufrechterhaltung von Bedingungen verstanden, die eine Funktionsfähigkeit des kultivierten Hornhautimplantates schafft oder gewährleistet, welche so weit wie möglich der Funktionsfähigkeit einer gesunden Hornhaut entspricht.

Im Zusammenhang mit der vorliegenden Erfindung wird unter einer "Funktionsfähigkeit einer gesunden Hornhaut" verstanden, dass die Hornhaut die Funktion einer gesunden Hornhaut aufweist, das heißt insbesondere dem Hornhautträger ein scharfes Sehen ermöglicht. Zudem muss die Zelldichte des Endothels über 2000 Zellen pro cm² liegen werden, da es sonst zu einem Quellvorgang des Korneagewebes kommt.

Im Zusammenhang mit der vorliegenden Erfindung wird unter einer "Kultivierung" auch eine Lagerung verstanden.

Im Zusammenhang mit der vorliegenden Erfindung wird unter einem Kulturmedium insbesondere ein flüssiges Kulturmedium, insbesondere ein Nährmedium, insbesondere ein flüssiges Nährmedium, insbesondere eine Nährflüssigkeit, insbesondere eine Nährflüssigkeit, die der Tränenflüssigkeit entspricht und vorzugsweise Tränenflüssigkeit selbst verstanden.

In einer besonders bevorzugten Ausführungsform der vorliegenden Erfindung ist die zumindest bereichsweise gewölbte Oberfläche des Bodenteils eine Oberfläche mit einer Wölbung, die der Wölbung einer natürlicherweise vorkommenden Hornhaut entspricht.

In einer bevorzugten Ausführungsform der vorliegenden Erfindung können ein oder mehrere Kanäle, die in Fluidzusammenhang mit dem zweiten Flüssigkeitskreislauf stehen, in der gewölbten Oberfläche des Bodenteils vorgesehen sein.

In einer besonders bevorzugten Ausführungsform weist der erfindungsgemäße Bioreaktorsystem mehr als eine Flüssigkeitspumpe, insbesondere zwei Pumpen, auf, nämlich eine dem ersten Flüssigkeitskreislauf und eine dem zweiten Flüssigkeitskreislauf zugeordnete Pumpe.

Die vorliegende Erfindung betrifft in einer besonders bevorzugten Ausführungsform ein erfindungsgemäßes Bioreaktorsystem, wobei die Fixiervorrichtung ein senkrecht in der Kulturkammer angeordneter Hohlzylinder, insbesondere ein Ring, ist.

Erfindungsgemäß bevorzugt wird ein Hohlzylinder, insbesondere Ring, eingesetzt, dessen Außenumfang und Durchmesser geringer, vorzugsweise etwas geringer, insbesondere mindestens 1%, vorzugsweise mindestens 2% geringer als der Innenumfang und der Durchmesser der Kulturkammer ist, sodass der Hohlzylinder in bevorzugter Ausführungsform passgenau in die Kulturkammer eingeführt und unmittelbar an der Seitenwand angrenzend platziert werden kann. Erfindungsgemäß bevorzugt kann ein solcher Hohlzylinder auch ein Bodenteil mit einer zentral gelegenen Öffnung aufweisen, wobei die Größe, Form und Position der Öffnung der Größe, Form und Position der gewölbten Oberfläche des Bodenteils entspricht. Der Bodenteil dieses Hohlzylinders kann das Hornhautimplantat auf dem Randbereich des Bodenteils um dessen Gesamtumfang fluiddicht fixieren.

Die vorliegende Erfindung betrifft in einer besonders bevorzugten Ausführungsform ein erfindungsgemäßes Bioreaktorsystem, wobei die Höhe des Hohlzylinders so bemessen ist, dass der Hohlzylinder bei Verschließen des Bioreaktorsystems mit dem Deckelelement bodenwärts gedrückt und so einen zwischen Randbereich der Oberfläche des Bodenteils und Unterseite der Fixiervorrichtung angeordneten Peripheriebereich eines Hornhautimplantats einklemmt. In besonders bevorzugter Ausführungsform entspricht die Höhe des Hohlzylinders der Höhe der mindestens einen Seitenwand.

In einer weiteren bevorzugten Ausführungsform kann die Fixiervorrichtung auch eine vollumfänglich an der Seiteninnenwand parallel zum Bodenteil verlaufende Spann- oder Klemmvorrichtung, insbesondere Klemmleiste sein, in der fluiddicht der Peripheriebereich des Hornhautimplantats fixiert, zum Beispiel eingeklemmt, wird.

Die vorliegende Erfindung betrifft in einer besonders bevorzugten Ausführungsform ein erfindungsgemäßes Bioreaktorsystem, wobei die Fixiervorrichtung zur Fixierung eines Hornhautimplantats, die Kulturkammer, die Flüssigkeitskreislaufsysteme oder Kombinationen davon aus Polyetheretherketon, PSU (Polysulfon), PPSU (Polyphenylsulfon), PTFE (Polytetrafluorethylen) oder Kombinationen davon, hergestellt sind.

Die vorliegende Erfindung betrifft in einer besonders bevorzugten Ausführungsform ein erfindungsgemäßes Bioreaktorsystem, wobei in dem ersten Flüssigkeitskreislauf ein erster Vorratsbehälter, insbesondere für ein flüssiges Kultur-, insbesondere Nährmedium, angeordnet ist.

Die vorliegende Erfindung betrifft in einer besonders bevorzugten Ausführungsform ein erfindungsgemäßes Bioreaktorsystem, wobei in dem zweiten Flüssigkeitskreislaufsystem ein zweiter Vorratsbehälter, insbesondere für ein flüssiges Kultur-, insbesondere Nährmedium, angeordnet ist.

Die vorliegende Erfindung betrifft in einer besonders bevorzugten Ausführungsform ein erfindungsgemäßes Bioreaktorsystem, wobei in dem ersten oder zweiten, vorzugsweise im zweiten, Flüssigkeitskreislaufsystem ein Drucksensor angeordnet ist.

Mittels des Drucksensors ist es möglich, den durch die mindestens eine Pumpe entstandenen hydrostatischen Druck zu messen und über den erfindungsgemäß bevorzugt vorgesehenen variablen Widerstand wunschgemäß zu regulieren.

Die vorliegende Erfindung betrifft in einer besonders bevorzugten Ausführungsform ein erfindungsgemäßes Bioreaktorsystem, wobei in dem ersten oder zweiten, vorzugsweise im zweiten, Flüssigkeitskreislaufsystem ein variabler Widerstand angeordnet ist.

Ein in einer besonders bevorzugt vorgesehenen Ausführungsform der vorliegenden Erfindung vorliegender variabler Widerstand, vorzugsweise im zweiten Flüssigkeitskreislaufsystem, erlaubt die Einstellung eines erwünschten hydrostatischen Drucks, insbesondere eines Druckes, der dem physiologischen Zustand im Augapfel entspricht, vorzugsweise ein Druck von 5 bis 40 mm Hg, vorzugsweise 7 bis 35 mm Hg, insbesondere 10 bis 20 mm Hg.

Die vorliegende Erfindung betrifft in einer besonders bevorzugten Ausführungsform ein erfindungsgemäßes Bioreaktorsystem, wobei der erste Vorratsbehälter, der zweite Vorratsbehälter oder beide Vorratsbehälter einen Sterilfilter zum Gasaustausch mit ihrer Umgebung, zum Beispiel mit Luft, CO₂, insbesondere Luft mit 5% CO₂-Zusatz, aufweisen.

Das vorliegende erfindungsgemäße Bioreaktorsystem kann auch als Modul eines Bioreaktorsystems ausgebildet sein, insbesondere eines Bioreaktorsystems, in dem die ersten und zweiten Flüssigkeitskreislaufsysteme mehr als eine Kulturkammer verbinden.

In besonders bevorzugter Ausführungsform ist das Bioreaktorsystem modular aufgebaut. Im Zusammenhang mit der vorliegenden Erfindung bedeutet der Ausdruck "modular", dass das Bioreaktorsystem als eine Baueinheit aus standardisierten Einheiten, also Modulen, der einzelnen Komponenten aufgebaut ist. Der modulare Aufbau ermöglicht es, einzelne Komponenten, wie die Flüssigkeitspumpe, Zu- und/oder Ableitungen der Flüssigkeitskreisläufe, den Deckel oder die Kulturkammer, leichter gegen Ersatzkomponenten auszutauschen, ohne in das Gesamtsystem eingreifen zu müssen.

Die vorliegende Erfindung betrifft auch Verfahren zur Kultivierung, zur Herstellung und zur Testung von Hornhautimplantaten und Hornhäuten.

Die vorliegende Erfindung betrifft in besonders bevorzugter Ausführungsform ein Verfahren zur Kultivierung von Hornhautimplantaten, umfassend die folgenden Schritte:
a) Bereitstellen mindestens eines Hornhautimplantats und eines erfindungsgemäßen Bioreaktorsystems,
b) Fixieren des mindestens einen Hornhautimplantats mittels der Fixiervorrichtung in dem Bioreaktorsystem und
c) Kultivieren des mindestens einen Hornhautimplantats in dem Bioreaktorsystem.

Die vorliegende Erfindung betrifft in besonders bevorzugter Ausführungsform ein Verfahren zur Testung von Agenzien an Hornhautimplantaten, umfassend die folgenden Schritte:
i) Bereitstellen mindestens eines Hornhautimplantats und eines erfindungsgemäßen Bioreaktorsystems,
ii) Fixieren des mindestens einen Hornhautimplantats mittels der Fixiervorrichtung in dem Bioreaktorsystem,
iii) Kultivieren des mindestens einen Hornhautimplantats in dem Bioreaktorsystem unter Zugabe des zu testenden Agenz und
iv) Analysieren der Wirkung des zu testenden Agenz auf das mindestens eine Hornhautimplantat.

Die vorliegende Erfindung betrifft in besonders bevorzugter Ausführungsform Verfahren der vorliegenden Erfindung zur Kultivierung von Hornhautimplantaten und Testung von Agenzien an Hornhautimplantaten, wobei im ersten Flüssigkeitskreislaufsystem dem Hornhautimplantat ein Kultur-, insbesondere Nährmedium, kontinuierlich oder vorzugsweise periodisch zugeführt wird.

Die vorliegende Erfindung betrifft in besonders bevorzugter Ausführungsform Verfahren der vorliegenden Erfindung zur Kultivierung von Hornhautimplantaten und Testung von Agenzien an Hornhautimplantaten, wobei im zweiten Flüssigkeitskreislaufsystem dem Hornhautimplantat kontinuierlich ein Nährmedium zugeführt wird.

Die vorliegende Erfindung betrifft in besonders bevorzugter Ausführungsform Verfahren der vorliegenden Erfindung zur Kultivierung von Hornhautimplantaten und Testung von Agenzien an Hornhautimplantaten, wobei das im zweiten Flüssigkeitskreislaufsystem geführte Nährmedium unter Druck, insbesondere einem Druck von 5 bis 40 mm Hg, vorzugsweise 7 bis 35 mm Hg, insbesondere 10 bis 20 mm Hg, steht.

Die vorliegende Erfindung ermöglicht auch die Testung von zum Beispiel Chemikalien, Pharmazeutika oder Kosmetikprodukten. Die vorliegend ermöglichte Kultivierung von Hornhautimplantaten unter in-vivo-Bedingungen ermöglicht es, Langzeituntersuchungen besonders verlässlich und realistisch durchzuführen und reversible Schädigungen zu erkennen. Durch den besonders einfachen Zugang zu einem Experimentalauge unter in-vivo-Bedingung können in dem Bioreaktorsystem Chemikalien, Kosmetika, Pharmazeutika und dergleichen auf zum Beispiel Korrosion und Irritation getestet werden.

In erfindungsgemäß besonders bevorzugter Ausführungsform können die zu testenden Substanzen durch den Tropf- oder Zerstäuberapplikator in die Kulturkammer, insbesondere den oberen Kulturkammerinnenraum und damit auf die Hornhautoberseite, aufgebracht werden. In einer anderen Ausführungsform der vorliegenden Erfindung lassen sich zu testende Substanzen, sofern das Deckelelement nicht aufgebracht ist, von oben unmittelbar in die Kulturkammer einbringen.

Die vorliegende Erfindung betrifft in besonders bevorzugter Ausführungsform ein Verfahren zur Herstellung von Hornhautäquivalenten, umfassend die folgenden Schritte:
x) Bereitstellen mindestens eines Hornhautgrundgerüstes und eines erfindungsgemäßen Bioreaktorsystems,
y) Fixieren des mindestens einen Hornhautgrundgerüstes mittels der Fixiervorrichtung in dem Bioreaktorsystem und
z) Kultivieren des mindestens einen Hornhautgrundgerüstes in dem Bioreaktorsystem unter Zugabe von Biofaktoren zum Erhalt eines Hornhautäquivalentes.

Die vorliegende Erfindung betrifft insbesondere ein Verfahren zur Herstellung von Hornhautäquivalenten, wobei dem Hornhautgrundgerüst im ersten und zweiten Flüssigkeitskreislaufsystem gleiche oder verschiedene Nährmedien zugeführt werden. In besonders bevorzugter Ausführungsform wird dem Hornhautgrundgerüst im ersten Flüssigkeitskreislaufsystem während des Kultivierens ein Nährmedium periodisch zugeführt. In einer besonders bevorzugten Ausführungsform der vorliegenden Erfindung wird dem Hornhautgrundgerüst während des Kultivierens im zweiten Flüssigkeitskreislaufsystem kontinuierlich ein Nährmedium zugeführt, insbesondere unter Druck, insbesondere Druck von 5 bis 40 mm Hg, vorzugsweise 7 bis 35 mm Hg, insbesondere 10 bis 20 mm Hg.

In besonders bevorzugter Ausführungsform werden dem Hornhautgrundgerüst während des Kultivierens unter für einen Hornhautaufbau geeigneten Kulturbedingungen Biofaktoren, insbesondere Zellen, zum Beispiel Fibroblasten, Proteine, insbesondere Wachstumsfaktoren, Nucleinsäuren, Rezeptoren, Hormone, Vitamine, Mineralien und/oder Zellextrakte, insbesondere über den ersten und/oder zweiten Flüssigkeitskreislauf, hinzugegeben.

Erfindungsgemäß wird es daher ermöglicht, mithilfe des erfindungsgemäßen Bioreaktorsystems Hornhautäquivalente aufzubauen und zu kultivieren. Das fluidische System der vorliegenden Erfindung ermöglicht es, ein Hornhautgrundgerüst zum Aufbau einer Hornhaut mit Zellen zu besiedeln und weiter darin zu kultivieren. Dreidimensionale Gewebemodelle können demgemäß optimal unter dynamischen Bedingungen versorgt werden, wobei ein mechanischer Stress, der auf das Gewebe einwirkt, ein besonders natürliches Zellverhalten garantiert.

Die vorliegende Erfindung betrifft daher auch Hornhautäquivalente, herstellbar, insbesondere hergestellt, mittels eines erfindungsgemäßen Verfahrens zur Herstellung von Hornhautäquivalenten.

Weitere vorteilhafte Ausgestaltungen ergeben sich aus den Unteransprüchen.

Die Erfindung wird anhand der folgenden, nicht-einschränkenden Beispiele und der dazugehörigen Figuren näher erläutert.

Die Figuren zeigen:
Figur 1: zeigt schematisch den Aufbau eines Bioreaktorsystems der vorliegenden Erfindung mit einem im Deckelelement angeordneten Zerstäuberapplikator.
Figur 2: zeigt schematisch eine weitere Ausführungsform eines erfindungsgemäßen Bioreaktorsystems mit einem in der Seitenwand angeordneten Zerstäuberapplikator.
Figur 3: zeigt schematisch eine Aufsicht auf eine erfindungsgemäße Kulturkammer ohne Deckel und ohne eingebrachtes Hornhautimplantat.
Figur 4: zeigt schematisch den Aufbau des Bodenteils eines erfindungsgemäßen Bioreaktorsystems.
Figur 5: zeigt schematisch den Aufbau des Bodenbereichs eines alternativen erfindungsgemäßen Bioreaktorsystems ohne Darstellung der Flüssigkeitskreisläufe.
Figur 6: zeigt einen fotografischen Vergleich zwischen zwei porcinen Hornhäuten, die gemäß Figur 6A mittels einer statischen Kultur und gemäß Figur 6B mittels der erfindungsgemäßen Verfahrensweise kultiviert wurden.

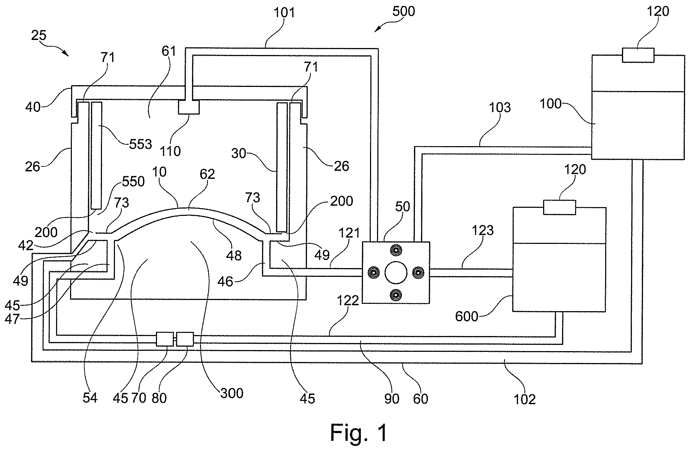

Beispiel 1: Figur 1 zeigt den schematischen Aufbau eines fluidischen Systems der vorliegenden Erfindung.

Das in Figur 1 dargestellte Bioreaktorsystem 500 befindet sich in einem hier nicht dargestellten Zell-Inkubator bei einer Temperatur von 32°C und einer mit 5% CO₂ versetzten Luft. Dargestellt ist ein erfindungsgemäßes Bioreaktorsystem 500, in den bereits ein Hornhautimplantat 10 eingebracht und fixiert wurde. Figur 1 zeigt die Kulturkammer 25 des Bioreaktorsystems 500 mit einem oben angeordneten Deckelelement 40, einer umlaufenden Seitenwand 26 der in Aufsicht gesehenen im Wesentlichen kreisrunden Kulturkammer 25 und einem unten angeordneten Bodenteil 45. Der Bodenteil 45 ist integral und einstückig mit der Seitenwand 26 ausgebildet und umfasst einen nach oben hin eben ausgebildeten Randbereich 49 sowie einen Zentralbereich 54. Der Zentralbereich 54 ist in seinem Zentrum, das heißt seinem Kernbereich 300, so ausgebildet, dass er zum Deckelelement 40 hin gewandt eine gewölbte Oberfläche 48 aufweist. Darüber hinaus befinden sich im Zentralbereich 54 Durchlassöffnungen, nämlich eine erste Durchlassöffnung 46 und eine zweite Durchlassöffnung 47, die den Innenraum der Kulturkammer 25 mit der Umgebung der Kulturkammer, insbesondere Zu- und Ableitungen des zweiten Flüssigkeitskreislaufs 90, verbinden. Die Wölbung 48 des Kernbereichs des Bodenteils 45 entspricht in Geometrie und Ausmaßen der Wölbung einer natürlicherweise vorkommenden Hornhaut.

In Figur 1 ist ferner die Fixiervorrichtung 30 dargestellt, die als Hohlzylinder, insbesondere Ring, mit einem Umfang und einem Durchmesser ausgebildet ist, der geringer, vorzugsweise etwas geringer, als der Umfang und Durchmesser des Innenraums der Kulturkammer 25 ist. Die Fixiervorrichtung ist senkrecht, das heißt mit ihren Öffnungen nach oben und nach unten gerichtet, vollumfänglich nahe der Seitenwand 26 des Kulturkammerinnenraums angeordnet. Die Höhe der Seitenwand 553 des Hohlzylinders ist so bemessen, dass sie der Höhe der Seitenwand 26 des Bioreaktorsystems 500 entspricht. Sofern, wie in Figur 1 dargestellt, ein Hornhautimplantat 10 zwischen Unterseite 200 der Fixiervorrichtung 30 und Randbereich 49 des Bodenteils 45 eingeklemmt wird, steht der Hohlzylinder etwas über die Oberkante 71 des Seitenrandes 26 hinaus und wird, sobald das Deckelelement 40 auf die Kulturkammer aufgesetzt, zum Beispiel aufgeschraubt, und fixiert wird, nach unten auf den eingeklemmten Peripheriebereich 73 des Hornhautimplantats 10 gedrückt. Das Hornhautimplantat 10 wird auf diese Weise fluiddicht zwischen dem Randbereich 49 des Bodenteils 45 und der Unterseite 200 der Fixiervorrichtung 30 fixiert, sodass sich ein oberhalb des fixierten Hornhautimplantats 10 befindlicher und durch dieses Hornhautimplantat von einem unteren Kulturkammerinnenraum 62 fluiddicht getrennt vorliegender oberer Kulturkammerinnenraum 61 ausbildet.

Dargestellt ist auch ein erster Flüssigkeitskreislauf 60, umfassend eine Leitung 101 von der mindestens einen Pumpe 50 zu dem Zerstäuberapplikator 110, einen oberhalb des fixierten Hornhautimplantats 10 liegenden oberen Kulturkammerinnenraum 61, eine Leitung 102 von der Durchlassöffnung 42 zu einem Vorratsbehälter 100 für ein Medium und eine Leitung 103 von dem Kulturmediumbehälter 100 zu der mindestens einen Pumpe 50. Die Durchlassöffnung 42 steht über eine als teilumfängliche Seitenwandverkürzung ausgeführte Öffnung 550 im unteren Bereich der Fixiervorrichtung 30 mit dem oberen Kulturkammerinnenraum 61 in Fluidverbindung. Diese Öffnung 550 befindet sich lediglich in genau dem der Durchlassöffnung 42 zugeordneten Bereich der Fixiervorrichtung 30, die ansonsten mit ihrer Unterseite 200 fluiddicht das Hornhautimplantat 10 auf den Randbereich 49 des Bodenteils 45 presst. Der erste Flüssigkeitskreislauf 60 ermöglicht es der mindestens einen Pumpe 50, Kulturmedium aus dem Kulturmediumsbehälter 100 über die Leitungen 103 und 101 zum Zerstäuberapplikator 110 zu transportieren. Im Zerstäuberapplikator 110 wird das Nährmedium auf die im oberen Reaktorinnenraum 61 befindliche Hornhaut 10 periodisch aufgebracht, beispielsweise vernebelt oder getropft, und fließt dann aus dem Kulturkammerinnenraum 61 durch die Durchlassöffnung 42 über die Leitung 102 zurück in den Kulturmediumbehälter 100. Der Kulturmediumbehälter 100 ist mit einem Sterilfilter 120 ausgestattet, der einen Gasaustausch mit einer mit 5% CO₂ angereicherten Luft gestattet.

Dargestellt ist auch der zweite Flüssigkeitskreislauf 90, umfassend eine Leitung 121 von der mindestens einen Pumpe 50 zur ersten Durchlassöffnung 46 im Randbereich 49 des Bodenteils 45 der Kulturkammer 25, einen unterhalb des fixierten Hornhautimplantats 10 liegenden unteren Kulturkammerinnenraum 62, eine Leitung 122 von der Auslassöffnung 47 zu dem zweiten Vorratsbehälter 600 und eine Leitung 123 vom Vorratsbehälter 600 zu der mindestens einen Pumpe 50. In der Leitung 122 befinden sich am Eingang oder Ausgang des Reaktors ein Drucksensor 70 und ein variabler Widerstand 80 mittels derer der Druck in dem zweiten Flüssigkeitskreislaufsystem 90 gemessen und auf einen hydrostatischen Druck von 10 bis 20 mm Quecksilbersäule eingestellt wird. Der sich in dem zweiten Flüssigkeitskreislauf 90 ausbildende hydrostatische Druck erlaubt es dem sich im zweiten Flüssigkeitskreislaufsystem 90 befindlichen Medium unter dem Hornhautimplantat 10 im zweiten Flüssigkeitskreislaufsystem 90 hindurchzufließen und so von dem zweiten Vorratsbehälter 600 über die Pumpe 50 durch die Einlassöffnung 46 in den Innenraum 62 der Kulturkammer 25 unterhalb des Hornhautimplantats 10 und durch die Auslassöffnung 47 zurück in den Vorratsbehälter 600 zu fließen. Alternativ kann das zweite Flüssigkeitskreislaufsystem auch ohne Pumpe 50 betrieben werden, wenn der Vorratsbehälter 600 oberhalb des Reaktors steht, auf den Drucksensor 70 und den Widerstand 80 kann dann verzichtet werden.

Das erste Flüssigkeitskreislaufsystem 60 führt periodisch der Hornhautoberseite tropfenweise oder in vernebelter Form Nährmedium zu. Der zweite Flüssigkeitskreislauf 90 imitiert durch den dort eingestellten hydrostatischen Druck den physiologisch vorliegenden Augeninnendruck und führt der Hornhautunterseite Nährmedium zu. Auf diese Art und Weise werden zwei unterschiedliche der in-vivo-Situation angenäherte oder identische Bedingungen für die Hornhautober- und -unterseite bereitgestellt.

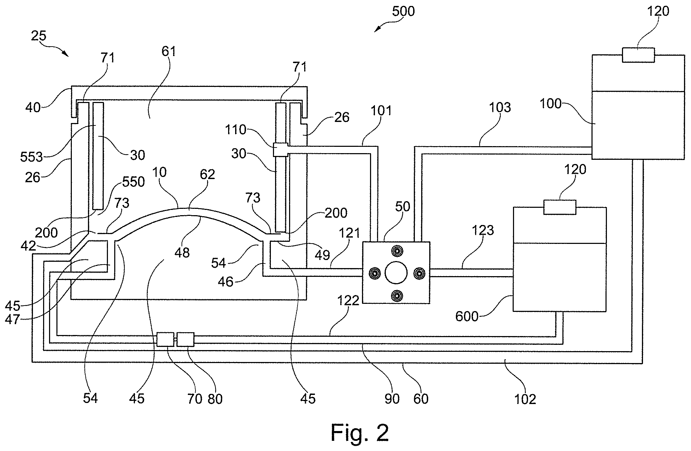

Figur 2 zeigt eine Vorrichtung gemäß der Figur 1, wobei gleiche Bezugszeichen auch gleiche Bedeutung haben. Im Unterschied zu Figur 1 befindet sich der Zerstäuberapplikator 110 nicht im Deckelelement 40, sondern in der Seitenwand 26.

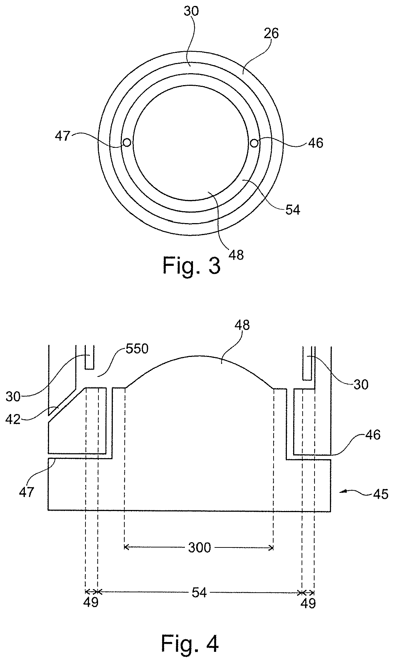

Figur 3 zeigt eine Aufsicht auf eine Kulturkammer des erfindungsgemäßen Bioreaktorsystems ohne eingebrachtes Hornhautimplantat. Dargestellt ist die umlaufende Seitenwand 26, die nah an dieser Seitenwand 26 angeordnete, in Form eines Hohlzylinders ausgeführte Fixiervorrichtung 30 sowie der Zentralbereich 54 des Bodenteils mit den beiden Auslassöffnungen 46 und 47 sowie die im Kernbereich 300 des Zentralbereichs 54 ausgeführte Wölbung 48.

Figur 4 verdeutlicht den Aufbau des Bodenteils des erfindungsgemäßen Reaktors. Dargestellt ist der Bodenteil 45 mit seinem peripheren Randbereich 49, der nach oben hin eben ausgebildet ist und keine Durchlassöffnungen aufweist sowie der Zentralbereich 54, der die Durchlassöffnungen 46 und 47 aufweist sowie in seinem Kernbereich 300 eine gewölbte Oberfläche 48 aufweist.

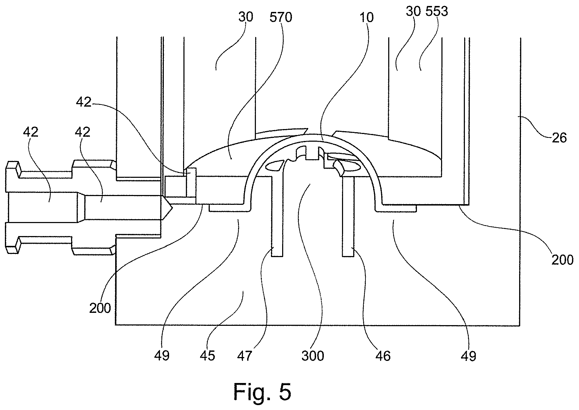

Figur 5 zeigt in schematischer Schnittdarstellung den Aufbau des Bodenbereiches eines alternativen Bioreaktorsystems, das im Wesentlichen dem in Figuren 1 und 2 dargestellten Bioreaktorsystem 500 entspricht, wobei die Fixiervorrichtung 30 als ein U-förmiges Profil ausbildender kreisrunder Hohlzylinder mit einer Seitenwand 553 und einem Bodenteil 570 ausgeführt ist. Die Fixiervorrichtung 30 weist in ihrem zentralen Bodenbereich eine Öffnung auf, die in ihrer Position, Form und Größe dem Kernbereich 300 des Zentralbereichs 54 des Bodenteils 45, also dem gewölbten Bereich des Bodenteils 45, entspricht. Wie in den Figuren 1 und 2 dargestellt, fixiert die Unterseite 200 der Fixiervorrichtung 30 das Hornhautimplantat 10 im Randbereich 49 des Bodenteils 45 und dichtet den oberen Kulturkammerinnenraum 61 vom unteren Kulturkammerinnenraum 62 ab. Im peripheren Bodenteil 570 der mit dem U-förmigen Profil ausgebildeten Fixiervorrichtung 30 befindet sich eine Öffnung 550, die Fluidverbindung zur Auslassöffnung 42 herstellt.

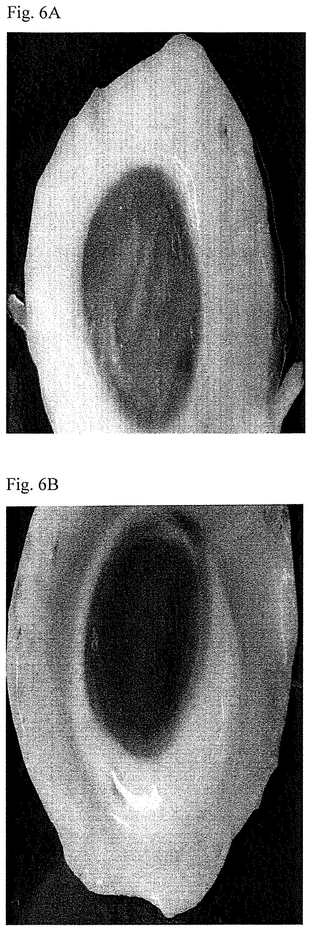

Mittels der erfindungsgemäßen Vorrichtung gemäß Figur 1 wurden porcine Hornhäute gelagert und verglichen mit porcinen Hornhäuten, die mittels einer statischen Kultur gelagert wurden. Gemäß Figur 6 zeigen sich deutliche Unterschiede. Die in dem erfindungsgemäßen System kultivierten Hornhäute zeigen keinerlei Anzeichen für Schwellungen oder Trübungen, während mit der statischen Kultur kultivierte Hornhäute stark getrübt sind.

## Patentansprüche

1. Bioreaktorsystem zur Kultivierung von Hornhautimplantaten, umfassend
a) eine Kulturkammer (25) mit einem Deckelelement (40), mindestens einer Seitenwand (26), mindestens einem an der Seitenwand (26) oder dem Deckelelement (40) angeordneten Tropf- oder Zerstäuberapplikator (110), mindestens einer Auslassöffnung (42) und einem Bodenteil (45), welches Bodenteil (45) eine zum Deckelelement (40) hin zumindest bereichsweise gewölbte Oberfläche (48) und mindestens eine erste und eine zweite Durchlassöffnungen (46, 47) aufweist,
b) mindestens eine Flüssigkeitspumpe (50),
c) ein erstes und zweites, jeweils mit der mindestens einen Flüssigkeitspumpe (50) in Verbindung stehendes Flüssigkeitskreislaufsystem (60, 90), wobei das erste Flüssigkeitskreislaufsystem (60) den mindestens einen Tropf- oder Zerstäuberapplikator (110) mit der mindestens einen Auslassöffnung (42) und das zweite Flüssigkeitskreislaufsystem (90) die erste mit der zweiten Durchlassöffnung (46, 47) verbindet und
d) mindestens eine in der Kulturkammer (25) relativ zur Seitenwand (26) beweglich angeordnete Fixiervorrichtung (30) zur vollumfänglichen Fixierung des Peripheriebereiches eines parallel zum Bodenteil (45) angeordneten Hornhautimplantats (10) an der mindestens einen Seitenwand (26) oder einem Randbereich (49) des Bodenteils (45), wobei durch die Fixierung des Peripheriebereichs des Hornhautimplantats eine fluiddichte Abtrennung des ersten Flüssigkeitskreislaufsystem (60) vom zweiten Flüssigkeitskreislaufsystem (90) erreicht wird.

2. Bioreaktorsystem nach Anspruch 1, wobei die Durchflussöffnungen (46, 47) im Zentralbereich (54) des Bodenteils (45) angeordnet sind.

3. Bioreaktorsystem nach einem der vorhergehenden Ansprüche, wobei die Vorrichtung (30) ein senkrecht in der Kulturkammer (25) angeordneter Hohlzylinder ist.

4. Bioreaktorsystem nach Anspruch 3, wobei die Höhe des Hohlzylinders so bemessen ist, dass er bei Verschließen des Bioreaktorsystems mit dem Deckelelement (40) einen zwischen Randbereich (49) des Bodenteils (45) und Fixiervorrichtung (30) angeordneten Peripheriebereich eines Hornhautimplantats einklemmt.

5. Bioreaktorsystem nach einem der vorhergehenden Ansprüche, wobei die Fixiervorrichtung zur Fixierung eines Hornhautimplantats (30), die Kultivierkammer (25), die Flüssigkeitskreislaufsysteme (60, 90) oder Kombinationen davon aus Polyetheretherketon, Polysulfon, Polyphenylsulfon, Polytetrafluorethylen oder Kombinationen davon hergestellt sind.

6. Bioreaktorsystem nach einem der vorhergehenden Ansprüche, wobei dem ersten Flüssigkeitskreislauf (60) ein Vorratsbehälter (100) zugeordnet ist.

7. Bioreaktorsystem nach einem der vorhergehenden Ansprüche, wobei in dem zweiten Flüssigkeitskreislaufsystem (90) ein zweiter Vorratsbehälter (600) angeordnet ist.

8. Bioreaktorsystem nach einem der vorhergehenden Ansprüche, wobei in dem zweiten Flüssigkeitskreislaufsystem (90) ein Drucksensor (70) angeordnet ist.

9. Bioreaktorsystem nach einem der vorhergehenden Ansprüche, wobei in dem zweiten Flüssigkeitskreislaufsystem (90) ein variabler Widerstand (80) angeordnet ist.

10. Bioreaktorsystem nach einem der vorhergehenden Ansprüche, wobei der erste Vorratsbehälter (100), der zweite Vorratsbehälter (600 oder beide Vorratsbehälter einen Sterilfilter (120) zum Gasaustausch mit ihrer Umgebung aufweisen.

11. Verfahren zur Kultivierung von Hornhautimplantaten, umfassend die folgenden Schritte:
a) Bereitstellen mindestens eines Hornhautimplantats und eines Bioreaktorsystems nach einem der Ansprüche 1 bis 10,
b) Fixieren des mindestens einen Hornhautimplantats mittels der Fixiervorrichtung (30) in dem Bioreaktorsystem und
c) Kultivieren des mindestens einen Hornhautimplantats in dem Bioreaktorsystem.

12. Verfahren zur Testung von Agenzien an Hornhautimplantaten, umfassend die folgenden Schritte:
i) Bereitstellen mindestens eines Hornhautimplantats und eines Bioreaktorsystems nach einem der Ansprüche 1 bis 10,
ii) Fixieren des mindestens einen Hornhautimplantats mittels der Fixiervorrichtung (30) in dem Bioreaktorsystem,
iii) Kultivieren des mindestens einen Hornhautimplantats in dem Bioreaktorsystem unter Zugabe des zu testenden Agenz und
iv) Analysieren der Wirkung des zu testenden Agenz auf das mindestens eine Hornhautimplantat.

13. Verfahren zur Herstellung von Hornhautäquivalenten, umfassend die folgenden Schritte:
x) Bereitstellen mindestens eines Hornhautgrundgerüstes und eines Bioreaktorsystems nach einem der Ansprüche 1 bis 10,
y) Fixieren des mindestens einen Hornhautgrundgerüstes mittels der Fixiervorrichtung (30) in dem Bioreaktorsystem und
z) Kultivieren des mindestens einen Hornhautgrundgerüstes in dem Bioreaktorsystem unter Zugabe von Biofaktoren zum Erhalt eines Hornhautäquivalentes.

14. Verfahren nach einem der Ansprüche 11 bis 13, wobei im zweiten Flüssigkeitskreislaufsystem (90) dem Hornhautimplantat kontinuierlich ein Nährmedium zugeführt wird.

15. Verfahren nach einem der vorhergehenden Ansprüche 11 bis 14, wobei das im zweiten Flüssigkeitskreislaufsystem (90) zugeführte Nährmedium unter Druck steht.

16. Verfahren nach einem der vorhergehenden Ansprüche 11 bis 15, wobei im ersten Flüssigkeitskreislaufsystem (60) dem Hornhautimplantat ein Nährmedium periodisch zugeführt wird.

## Claims

1. Bioreactor system for cultivating corneal implants, comprising:
a) a culture chamber (25) having a cover element (40), at least one side wall (26), at least one drop applicator or atomiser applicator (110) arranged on the side wall (26) or cover element (40), at least one outlet opening (42), and a base part (45), which base part (45) has a surface (48), curved at least in regions towards the cover element (40), and at least a first and a second flow-through opening (46, 47),
b) at least one liquid pump (50),
c) a first and a second liquid circulation system (60, 90), each connected to the at least one liquid pump (50), the first liquid circulation system (60) connecting the at least one drop applicator or atomiser applicator (110) to the at least one outlet opening (42), and the second liquid circulation system (90) connecting the first and the second flow-through opening (46, 47), and
d) at least one fixing device (30), arranged movable in the culture chamber (25) relative to the side wall (26), for fixing the entire peripheral region of a corneal implant (10), arranged parallel to the base part (45), on the at least one side wall (26) or an edge region (49) of the base part (45), fluid-tight separation of the first liquid circulation system (60) from the second liquid circulation system (90) being achieved by fixing the peripheral region of the corneal implant.

2. Bioreactor system according to claim 1, wherein the flow-through openings (46, 47) are arranged in the central region (54) of the base part (45).

3. Bioreactor system according to any one of the preceding claims, wherein the device (30) is a hollow cylinder arranged vertically in the culture chamber (25).

4. Bioreactor system according to claim 3, wherein the height of the hollow cylinder is dimensioned in such a way that, when the bioreactor system is closed by the cover element (40), the cylinder grips a peripheral region of a corneal implant, which is arranged between the edge region (49) of the base part (45) and the fixing device (30).

5. Bioreactor system according to any one of the preceding claims, wherein the fixing device for fixing a corneal implant (30), the cultivation chamber (25), the liquid circulation systems (60, 90) or combinations thereof are made from polyetheretherketone, polysulfone, polyphenylsulfone, polytetrafluoroethylene or combinations thereof.

6. Bioreactor system according to any one of the preceding claims, wherein a reservoir container (100) is assigned to the first liquid circuit (60).

7. Bioreactor system according to any one of the preceding claims, wherein a second reservoir container (600) is arranged in the second liquid circulation system (90).

8. Bioreactor system according to any one of the preceding claims, wherein a pressure sensor (70) is arranged in the second liquid circulation system (90).

9. Bioreactor system according to any one of the preceding claims, wherein a variable resistor (80) is arranged in the second liquid circulation system (90).

10. Bioreactor system according to any one of the preceding claims, wherein the first reservoir container (100), the second reservoir container (600) or both reservoir containers comprise a sterile filter (120) for gas exchange with the environment thereof.

11. Method for cultivating corneal implants, comprising the following steps:
a) providing at least one corneal implant and a bioreactor system according to any one of claims 1 to 10,
b) fixing the at least one corneal implant by means of the fixing device (30) in the bioreactor system, and
c) cultivating the at least one corneal implant in the bioreactor system.

12. Method for testing agents on corneal implants, comprising the following steps:
i) providing at least one corneal implant and a bioreactor system according to any one of claims 1 to 10,
ii) fixing the at least one corneal implant by means of the fixing device (30) in the bioreactor system,
iii) cultivating the at least one corneal implant in the bioreactor system while adding the agent to be tested, and
iv) analysing the effect of the agent to be tested on the at least one corneal implant.

13. Method for producing cornea equivalents, comprising the following steps:
x) providing at least one cornea base structure and a bioreactor system according to any one of claims 1 to 10,
y) fixing the at least one cornea base structure by means of the fixing device (30) in the bioreactor system, and
z) cultivating the at least one cornea base structure in the bioreactor system while adding biofactors to obtain a cornea equivalent.

14. Method according to any one of claims 11 to 13, wherein a nutrient solution is supplied to the corneal implant continuously in the second liquid circulation system (90).

15. Method according to any one of the preceding claims 11 to 14, wherein the nutrient solution supplied in the second liquid circulation system (90) is under pressure.

16. Method according to any one of the preceding claims 11 to 15, wherein a nutrient medium is supplied to the corneal implant periodically in the first liquid circulation system (60).

## Revendications

1. Système de bioréacteur destiné à la culture d'implants cornéens, comprenant :
a) une chambre de culture (25) comportant un élément de couvercle (40), au moins une paroi latérale (26), au moins un applicateur de gouttes ou pulvérisateur (110) disposé sur la paroi latérale (26) ou l'élément de couvercle (40), au moins un orifice de sortie (42) et une partie de fond (45), laquelle partie de fond (45) présente une surface (48) au moins sur certaines parties bombée vers l'élément de couvercle (40) et au moins un premier et un second orifice de passage (46, 47),
b) au moins une pompe à liquide (50),
c) un premier et un second système de circuit de liquide (60, 90) se trouvant respectivement en liaison avec l'au moins une pompe à liquide (50), le premier système de circuit de liquide (60) reliant l'au moins un applicateur de gouttes ou pulvérisateur (110) à l'au moins un orifice de sortie (42) et le second système de circuit de liquide (90) reliant le premier et le second orifice de passage (46, 47) entre eux, et
d) au moins un dispositif de fixation (30) disposé dans la chambre de culture (25) de manière mobile par rapport à la paroi latérale (26) pour la fixation sur toute sa circonférence de la zone périphérique d'un implant cornéen (10) disposé parallèlement à la partie de fond (45) à l'au moins une paroi latérale (26) ou une zone de bord (49) de la partie de fond (45), la fixation de la zone périphérique de l'implant cornéen permettant d'obtenir une séparation étanche aux fluides entre le premier système de circuit de liquide (60) et le second système de circuit de liquide (90).

2. Système de bioréacteur selon la revendication 1, dans lequel les orifices de passage (46, 47) sont disposés dans la zone centrale (54) de la partie de fond (45).

3. Système de bioréacteur selon l'une des revendications précédentes, dans lequel le dispositif (30) est un cylindre creux disposé verticalement dans la chambre de culture (25).

4. Système de bioréacteur selon la revendication 3, dans lequel la hauteur du cylindre creux est dimensionnée de telle sorte qu'il coince une zone périphérique d'un implant cornéen disposé entre la zone de bord (49) de la partie de fond (45) et le dispositif de fixation (30) lors de la fermeture du système de bioréacteur avec l'élément de couvercle (40).

5. Système de bioréacteur selon l'une des revendications précédentes, dans lequel le dispositif de fixation (30) pour la fixation d'un implant cornéen, la chambre de culture (25), les systèmes de circuit de liquide (60, 90) ou des combinaisons de ceux-ci sont fabriqués en polyétheréthercétone, polysulfone, polyphénylsulfone, polytétrafluoroéthylène ou des combinaisons de ceux-ci.

6. Système de bioréacteur selon l'une des revendications précédentes, dans lequel un réservoir (100) est associé au premier circuit de liquide (60).

7. Système de bioréacteur selon l'une des revendications précédentes, dans lequel un second réservoir (600) est disposé dans le second système de circuit de liquide (90).

8. Système de bioréacteur selon l'une des revendications précédentes, dans lequel un capteur de pression (70) est disposé dans le second système de circuit de liquide (90).

9. Système de bioréacteur selon l'une des revendications précédentes, dans lequel une résistance variable (80) est disposée dans le second système de circuit de liquide (90).

10. Système de bioréacteur selon l'une des revendications précédentes, dans lequel le premier réservoir (100), le second réservoir (600) ou les deux réservoirs comportent un filtre stérile (120) pour l'échange gazeux avec leur environnement.

11. Procédé de culture d'implants cornéens, comprenant les étapes suivantes consistant à :
a) fournir au moins un implant cornéen et un système de bioréacteur selon l'une des revendications 1 à 10,
b) fixer l'au moins un implant cornéen dans le système de bioréacteur au moyen du dispositif de fixation (30), et
c) cultiver l'au moins un implant cornéen dans le système de bioréacteur.

12. Procédé de test d'agents sur des implants cornéens, comprenant les étapes suivantes consistant à :
i) fournir au moins un implant cornéen et un système de bioréacteur selon l'une des revendications 1 à 10,
ii) fixer l'au moins un implant cornéen dans le système de bioréacteur au moyen du dispositif de fixation (30),
iii) cultiver l'au moins un implant cornéen dans le système de bioréacteur en ajoutant l'agent à tester, et
iv) analyser l'effet de l'agent à tester sur l'au moins un implant cornéen.

13. Procédé de fabrication d'équivalents cornéens, comprenant les étapes suivantes consistant à :
x) fournir au moins une structure de base de cornée et un système de bioréacteur selon l'une des revendications 1 à 10,
y) fixer l'au moins une structure de base de cornée dans le système de bioréacteur au moyen du dispositif de fixation (30), et
z) cultiver l'au moins une structure de base de cornée dans le système de bioréacteur en ajoutant des biofacteurs pour obtenir un équivalent cornéen.

14. Procédé selon l'une des revendications 11 à 13, dans lequel, dans le second système de circuit de liquide (90), l'implant cornéen est alimenté en continu en milieu de culture.

15. Procédé selon l'une des revendications précédentes 11 à 14, dans lequel le milieu de culture alimenté dans le second système de circuit de liquide (90) est sous pression.

16. Procédé selon l'une des revendications précédentes 11 à 15, dans lequel, dans le premier système de circuit de liquide (60), l'implant cornéen est alimenté périodiquement en milieu de culture.
